# EUROPÄISCHE PATENTSCHRIFT

(11) **EP 0 665 027 B1**
(45) Veröffentlichungstag und Bekanntmachung des Hinweises auf die Patenterteilung: **15.12.1999**
(21) Anmeldenummer: 94101290.8
(22) Anmeldetag: 28.01.1994
(51) Int. Cl.: A61M 5/145

(54) **Umrüstsatz für einen Injektionsautomaten zur Verwendung mit Spritzen mit unterschiedlichen Durchmessern**
Adaptor for an injector for receiving syringes of different diameters
Adapteur pour un injecteur pour l'utilisation de seringues de diamètres différents

(43) Veröffentlichungstag der Anmeldung: 02.08.1995
(73) Patentinhaber: MALLINCKRODT MEDICAL GmbH, 53773 Hennef/Sieg (DE)
(72) Erfinder: Müller-Späth, Reinhard,Diplom-Volkswirt, D-53804 Much (DE)
(74) Vertreter: Kraus, Walter, Dr.

(56) Entgegenhaltungen:
- EP-A- 0 080 037
- EP-A- 0 346 950
- US-A- 3 623 474
- US-A- 3 813 012
- US-A- 5 279 569

## Beschreibung

Die Erfindung betrifft einen Umrüstsatz für einen Injektionsautomaten zum intravasalen Injizieren von parenteralen Lösungen, wie beispielsweise von Röntgenkontrastmittellösungen, aus Hochdruckinjektionsspritzen, wobei der umzurüstende Injektionsautomat folgendes aufweist:
(a) einen Rezipienten zum lösbaren Festhalten einer Hochdruckinjektionsspritze, der einen Aufnahmekopf besitzt, welcher mit einer zylindrischen Aufnahmeführung zum engen Umschließen des distalen Endbereichs des Hochdruckinjektionsspritzenkörpers und mit einer radialen Ausnehmung für die im wesentlichen drehfeste Aufnahme eines von dem distalen Ende des Hochdruckinjektionsspritzenkörpers vorstehenden Umfangsrandes versehen ist; und
(b) eine kraftgetrieben ein- und ausfahrbare Spindelstange, die zum Verschieben des Kolbens der Hochdruckinjektionsspritze an ihrem freien Ende einen lösbar daran befestigten Kolbenaufnehmer aufweist, welcher einen Ankopplungskopf zum Ankoppeln desselben an den Kolben der Hochdruckinjektionsspritze hat.

Zum intravasalen Injizieren von parenteralen Lösungen, insbesondere von Röntgenkontrastmittellösungen auf dem Gebiet der Phlebographie, d.h. der röntgenologischen Aufnahme der Beinvenen für die Thrombosediagnose, sowie auf dem umfangreichen Gebiet der Computertomographie, werden Injektionsautomaten verwendet, um relativ große Volumina im Maximalbereich von etwa 100 bis 200 ml programmiert zu injizieren. Auf diese Weise lassen sich beliebige Injektionsprofile äußerst genau, in praktisch jeder gewünschten Form und in unkomplizierter Art realisieren, die den jeweiligen medizinischen Bedürfnissen entsprechen. Durch Abspeichern der verschiedenen Injektionsprofile stehen diese immer wieder für die jeweiligen unterschiedlichen Untersuchungen zur Verfügung. Beispielsweise lassen sich mit solchen Injektionsautomaten Förderraten der parenteralen Lösung von 0,1 bis 9,9 ml/s erreichen, die in 0,1 ml/s-Schritten wählbar sind, wobei das maximale Injektionsvolumen, das zwischen etwa 100 und 200 ml liegt, in Teilvolumina injiziert werden kann, die beispielsweise in 1 ml-Schritten wählbar sind. Die Injektion erfolgt hierbei mit einem relativ hohen Injektionsdruck, der mehrere bar bis mehrere zehn bar betragen kann, und der aus Sicherheitsgründen elektronisch begrenzt wird.

Die für solche Injektionsautomaten verwendeten Injektionsspritzen, die im allgemeinen ein relativ großes Volumen zwischen maximal 100 und 200 ml haben, werden wegen des verhältnismäßig hohen Injektionsdrucks, der damit angewandt wird, im klinischen Sprachgebrauch als sogenannte Hochdruckinjektionsspritzen bezeichnet.

Außer den Hochdruckinjektionsspritzen für die Injektionsautomaten werden in der medizinischen Praxis nach wie vor in einem unvergleichlich viel größeren Umfang übliche Handinjektionsspritzen verwendet, die im allgemeinen ein demgegenüber relativ kleines Volumen im Bereich von 1 bis 50 ml haben. In vielen Fällen wäre es sehr wünschenswert, mit solchen Handinjektionsspritzen automatische Injektionen durchzuführen. Jedoch sind diese Handinjektionsspritzen anders ausgebildet als die Hochdruckinjektionsspritzen, so daß sie nicht in die für die Hochdruckinjektionsspritzen vorgesehenen Injektionsautomaten passen und daher einen speziell für Handinjektionsspritzen ausgebildeten Injektionsautomaten erfordern, was einen ziemlich hohen Kostenaufwand bedingt.

Aus der US-A-3 623 474 ist ein Injektionsautomat bekannt, der ausschließlich für Hochdruckinjektionsspritzen bestimmt ist, wobei zwei Bereiche des Hochdrucks unterschieden werden, nämlich ein niedriger Hochdruckbereich bis etwa 10 bar und ein höherer Hochdruckbereich bis etwa 55 bar. Für beide Hochdruckbereiche werden baulich gleichartig ausgebildete Injektionsspritzen verwendet, die aber bei Anwendung des höheren Hochdruckbereichs in einen druckfesten Mantel eingelegt werden, der zusammen mit der Injektionsspritze auf den Injektionsautomaten aufgeschraubt wird. Die Verbindung der Zylindergriffplatte mit dem Gehäuse des Injektionsautomaten und einer die Zylindergriffplatte übergreifenden Überwurfmutter erfolgt dadurch, daß die Zylinderplatte zwischen zwei Dichtungsteilen eingeklemmt wird. Eine Verwendung des Injektionsautomaten für Handinjektionsspritzen mit gegenüber Hochdruckinjektionsspritzen unterschiedlich gestalteter Zylindergriffplatte ist in der US-A-3 623 474 überhaupt nicht vorgesehen und nicht einmal erwähnt.

Weiter ist in der US-A-5 279 569 ein Hochdruckinjektionsautomat beschrieben und dargestellt, bei dem die verwendeten Hochdruckinjektionsspritzen von vorn her in einen am Gehäuse des Hochdruckinjektionsautomaten befestigten druckfesten Mantel geschoben werden, wonach eine Druckkappe auf dem vorderen Ende des druckfesten Mantels befestigt wird. Auch in der US-A-5 279 569 ist ebensowenig wie in der US-A-3 623 474 eine Verwendung des Injektionsautomaten für Handinjektionsspritzen vorgesehen, und eine solche Verwendung ist zudem überhaupt nicht möglich, weil Handinjektionsspritzen eine speziell ausgebildete Zylindergriffplatte haben, während der Hochdruckinjektionsautomat nach der US-A-5 279 569 so ausgebildet ist, daß die dort verwendeten Injektionsspritzen keinerlei Zylindergriffsplatte besitzen dürfen, um von vorn her formschlüssig in den druckfesten Mantel einschiebbar zu sein.

Außerdem sind in der EP-A-0 080 037 A1 und der EP-A-0 346 950 A2 Hochdruckinjektionsautomaten und Hochdruckinjektionsspritzen beschrieben, jedoch sind in diesen Druckschriften keinerlei Möglichkeiten einer Umrüstung der Hochdruckinjektionsautomaten für Handinjektionsspritzen mit gegenüber den Hochdruckinjektionsspritzen unterschiedlich gestalteten Zylindergriffplatten offenbart oder angesprochen.

Aufgabe der vorliegenden Erfindung ist es insbesondere, eine Möglichkeit zur Verfügung zu stellen, die den technischen Aufwand für das automatische intravasale Injizieren von parenteralen Lösungen aus Handinjektionsspritzen wesentlich vermindert.

Diese Aufgabe wird erfindungsgemäß durch einen Umrüstsatz der eingangs genannten Art gelöst, der es ermöglicht, einen Injektionsautomaten für Hochdruckinjektionsspritzen wahlweise auch für die automatische Injektion mit Handinjektionsspritzen zu benutzen.

Der Umrüstsatz nach der vorliegenden Erfindung ist im einzelnen im Patentanspruch 1 und/oder 2 angegeben, und Weiterbildungen desselben sind in den Patentansprüchen 3 bis 12 charakterisiert.

Der radial von dem distalen Ende der Reduzierhülse vorstehende "Umfangsrand" umfaßt im Sinne der vorliegenden Erfindung bevorzugt die gesamte Stirnseite des distalen Endes der Reduzierhülse, also nicht nur den über den Hülsenkörper vorstehenden Teil, sondern auch einen dem Querschnittsbereich des Hülsenkörpers entsprechenden Teil:

Erfindungsgemäß umfaßt der Umrüstsatz zum Umrüsten des Injektionsautomaten für ein intravasales Injizieren aus Handinjektionsspritzen, der mit der vorliegenden Erfindung zur Verfügung gestellt wird, folgendes:
(1) wenigstens einen Umrüstrezipienten, bei dem die radiale Ausnehmung des Aufnahmekopfs außer einem ersten Ausnehmungsbereich für die im wesentlichen drehfeste Aufnahme des von dem distalen Ende eines Hochdruckinjektionsspritzenkörpers vorstehenden Umfangsrandes, einen zweiten Ausnehmungsbereich für die radialen Endbereiche der Flügel einer Handinjektionsspritze vorbestimmter Größe aufweist;
(2) wenigstens eine Reduzierhülse zur Verkleinerung des effektiven Innendurchmessers der zylindrischen Aufnahmeführung, umfassend
   (i) einen im wesentlichen zylindrischen Hülsenkörper, dessen Außendurchmesser dem Innendurchmesser der Aufnahmeführung (19) entspricht und dessen Innendurchmesser dem Außendurchmesser des Handinjektionsspritzenkörpers (5) entspricht, und
   (ii) einen radial von dem distalen Ende der Reduzierhülse vorstehenden Umfangsrand, welcher in der Ausnehmung des Aufnahmekopfes aufnehmbar ist und in welchem der Umfangsrand des distalen Endes der Handinjektionsspritze bis auf die Endbereiche der Flügel aufnehmbar ist; und
(3) wenigstens einen Umrüstkolbenaufnehmer, dessen radialer Maximaldurchmesser gleich dem oder kleiner als der Durchmesser des Kolbens der Handinjektionsspritze ist, und der mit einem Befestigungsmittel zum lösbaren Befestigen desselben an dem freien Ende der Spindelstange sowie mit einem Ankopplungskopf zum Ankoppeln des Kolbenaufnehmers an den Kolben einer Hochdruckinjektionsspritze und zum Drücken auf den Kolben der Handinjektionsspritze versehen ist.

Mit diesem Umrüstsatz gemäß der vorliegenden Erfindung kann ein Injektionsautomat für Hochdruckinjektionsspritzen in verhältnismäßig schneller, unkomplizierter und äußerst kostengünstiger Weise in einen für Handinjektionsspritzen benutzbaren Injektionsautomaten umgewandelt werden, so daß der Injektionsautomat wahlweise für Hochdruckinjektionsspritzen oder Handinjektionsspritzen verwendet werden kann.

In einer bevorzugten Ausführungsform ist der Umrüstsatz so ausgebildet, daß er für - gleichartig gestaltete, gegebenenfalls unterschiedlich lange - Handinjektionsspritzen von 30 ml und 50 ml und für Hochdruckinjektionsspritzen von 50 ml bis 200 ml verwendbar ist.

In einer anderen bevorzugten Ausführungsform umfaßt der Umrüstsatz mehrere Umrüstrezipienten und/oder mehrere Reduzierhülsen und/oder mehrere Umrüstkolbenaufnehmer derart, daß der Injektionsautomat damit für Handinjektionsspritzen, deren Injektionsspritzenkörper unterschiedliche Durchmesser haben, verwendbar gemacht werden kann, z.B. für alle oder einige ausgewählte Größen von Handinjektionsspritzen aus dem Größenbereich von 1 ml bis 50 ml.

Um einen gegenüber geringen Kräften, wie sie bei der Handhabung ausgeübt werden, verschiebungssicheren Sitz der Reduzierhülse in der zylindrischen Aufnahmeführung des Aufnahmekopfs des Rezipienten sicherzustellen, ist in einer bevorzugten Ausführungsform des erfindungsgemäßen Umrüstsatzes die Aufnahmeführung mit einem, vorzugsweise um den inneren Umfang derselben umlaufenden, elastischen Vorsprung versehen, und die Reduzierhülse weist auf deren Außenumfang eine diesem Vorsprung entsprechende Vertiefung auf; oder umgekehrt. Eine besonders einfache und praktische Gestaltung dieser Art wird dadurch erreicht, daß der elastische Vorsprung von einem O-Ring gebildet ist, der in eine auf dem Innenumfang der Aufnahmeführung oder der Reduzierhülse ausgebildeten Nut eingelegt ist.

Die Reduzierhülse ist vorzugsweise aus Kunststoff hergestellt, und zwar besonders bevorzugt aus einem elastischen Kunststoff, wie Polytetrafluorethylen, Polyester oder Polyamid.

Die vorstehend angegebenen sowie weitere Vorteile und Merkmale der Erfindung seien nachfolgend anhand einer besonders bevorzugten Ausführungsform eines erfindungsgemäßen Umrüstsatzes unter Bezugnahme auf die Figuren der Zeichnung näher erläutert; es zeigen:
Figur 1 eine im wesentlichen im Querschnitt und schematisch dargestellte Teilansicht eines Injektionsautomaten zum intravasalen Injizieren von parenteralen Lösungen aus Hochdruckinjektionsspritzen, der durch eine bevorzugte Ausführungsform eines Umrüstsatzes nach der Erfindung für das automatisch gesteuerte Injizieren solcher Lösungen aus Handinjektionsspritzen verwendbar gemacht ist;
Figur 2 einen Querschnitt durch den Aufnahmekopf eines Umrüstrezipienten, wie er in einer Ausführungsform eines erfindungsgemäßen Umrüstsatzes vorgesehen ist;
Figur 3 eine Aufsicht auf den Aufnahmekopf des Umrüstrezipienten der Figur 2 von unten, d.h. von der Seite her, von welcher die - vorzugsweise schon vorgefüllte - Handinjektionsspritze her in die Aufnahmeführung dieses Aufnahmekopfes eingeführt wird und die in Figur 1 oben ist;
Figur 4 einen Axialschnitt gemäß der Linie A-A in Figur 5 durch eine Ausführungsform einer Reduzierhülse eines erfindungsgemäßen Umrüstsatzes;
Figur 5 eine Aufsicht auf die Reduzierhülse der Figur 4 von oben, d.h. von der Seite her, welche den Umfangsrand aufweist, der von der Ausnehmung des Aufnahmekopfs des Umrüstrezipienten aufgenommen wird und der seinerseits die Zylindergriffplatte (distaler radialer Umfangsrand) der Handinjektionsspritze bis auf die radial darüber vorstehenden Endbereiche der Flügel aufnimmt;
Figur 6 einen Axialschnitt durch eine Ausführungsform eines Umrüstkolbenaufnehmers eines erfindungsgemäßen Umrüstsatzes gemäß der Linie B-B der Figur 7, jedoch ohne den inneren Konus und die Schraube der Figur 1 zu dessen Befestigung;
Figur 7 eine Aufsicht auf den Kolbenaufnehmer der Figur 6 von oben, d.h. von der Seite her, die beim Einbau der kraftgetriebenen ein- und ausfahrbaren Spindelstange des Injektionsautomaten zugewandt ist und in die der in Figur 1 gezeigte innere Konus eingeschraubt wird;
Figur 8 eine Aufsicht auf den Kolbenaufnehmer der Figur 6 von unten, d.h. von der Seite her, mit welcher er im umgerüsteten Zustand des Injektionsautomaten auf den Kolben der Handinjektionsspritze zur Verschiebung desselben drückt und an welcher er bei herausgenommener Reduzierhülse mit dem ein verdicktes Ende aufweisenden Zapfen, der am Kolben einer Hochdruckinjektionsspritze vorgesehen ist, lose so verbindbar ist, daß der Kolben der Hochdruckinjektionsspritze wahlweise durch die kraftgetriebene Spindelstange in einem Saug- oder Injektionshub betätigt werden kann;
Figur 9 einen Axialschnitt durch eine Hochdruckinjektionsspritze ohne den Kolben derselben, sowie eine Aufsicht auf das distale Ende derselben, welche die Form des von dem Hochdruckinjektionsspritzenkörper als Zylindergriffplatte radial vorstehenden Umfangsrands veranschaulicht; und
Figur 10 einen Axialschnitt durch eine Handinjektionsspritze ohne den Kolben derselben, sowie eine Aufsicht auf das distale Ende dieser Handinjektionsspritze, aus welcher die Form der als Umfangsrand ausgebildeten Zylindergriffplatte ersichtlich ist, die zwei radial entgegengesetzt gerichtete Flügel aufweist.

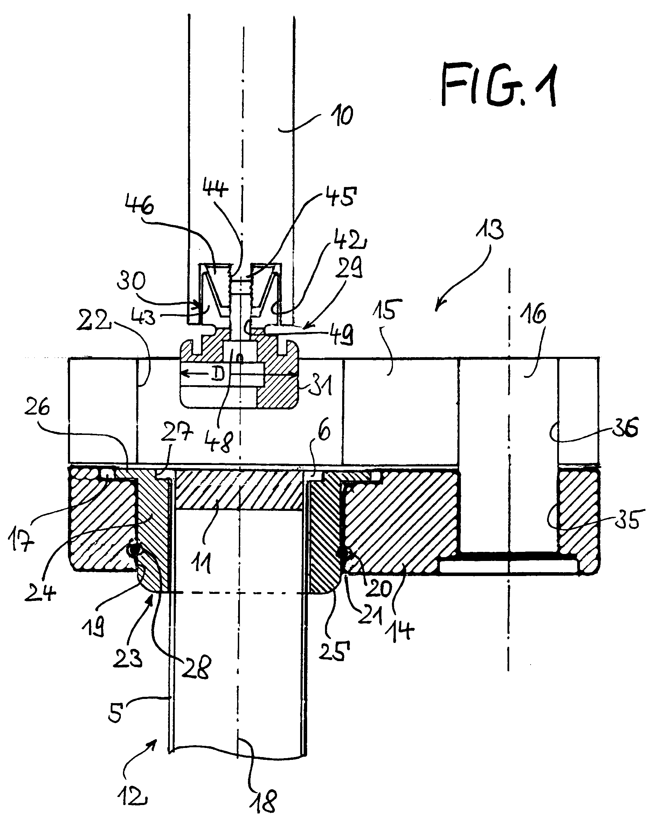

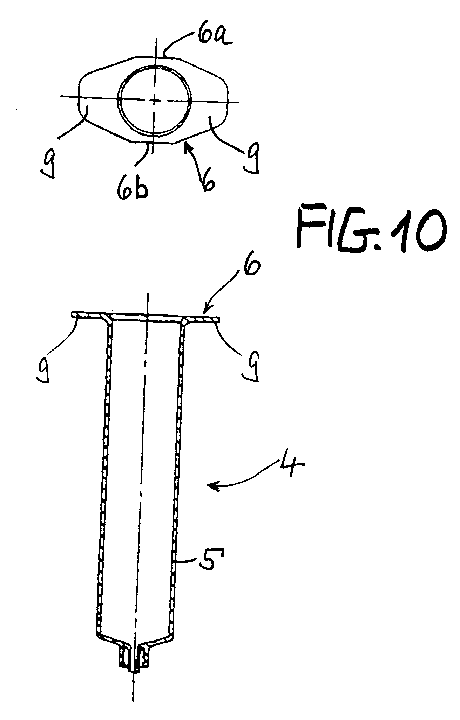

Bevor anhand der Figur 1 ein Injektionsautomat erläutert wird, der mittels eines erfindungsgemäßen Umrüstsatzes für die Benutzung des Injektionsautomaten zur automatischen Injektion aus Handinjektionsspritzen umgebaut ist, sei zunächst anhand der Figuren 9 und 10 der wesentliche Unterschied zwischen üblichen Handinjektionsspritzen und Hochdruckinjektionsspritzen, soweit er für die vorliegende Erfindung von besonderer Bedeutung ist, näher erläutert:
In Figur 9 ist ein üblicher Hochdruckinjektionsspritzenkörper 1 im Axialschnitt gezeigt, der einen Spritzenzylinder 2 und eine als radial davon vorstehender Umfangsrand 3 ausgebildete Zylindergriffplatte an seinem distalen Ende hat, und Figur 10 zeigt die entsprechenden Teile eines üblichen Handinjektionsspritzenkörpers 4, der einen Spritzenzylinder 5 hat, an dessen distalem Ende eine Zylindergriffplatte als radial nach außen vorstehender Umfangsrand 6 ausgebildet ist.

Die vorliegend interessierenden beiden wesentlichen Unterschiede zwischen dem Hochdruckinjektionsspritzenkörper 1 und dem Handinjektionsspritzenkörper 4 bestehen darin, daß der Spritzenzylinder 5 des Handinjektionsspritzenkörpers 4 einen kleineren Innen- und Außendurchmesser als der Spritzenzylinder 2 des Hochdruckinjektionsspritzenkörpers 1 hat, da ersterer für kleinere Nennvolumina als letzterer bestimmt ist. Dementsprechend ist der Außendurchmesser des Kolbens einer Handinjektionsspritze kleiner als derjenige des Kolbens einer Hochdruckinjektionsspritze. Weiterhin ist der die Zylindergriffplatte bildende Umfangsrand 6 des Handinjektionsspritzenkörpers 4 anders geformt als der als Zylindergriffplatte vorgesehene Umfangsrand 3 des Hochdruckinjektionsspritzenkörpers 1. Wie die Figur 9 zeigt, besteht der Umfangsrand 3 des Hochdruckinjektionsspritzenkörpers 2 aus einem Kreisring 7, der diametral gegenüberliegende Abflachungen 8 hat, die dazu dienen, den Umfangsrand 7 im wesentlichen drehfest in einer Ausnehmung des Rezipienten eines Injektionsautomaten anzuordnen, wobei es auch Ausführungsformen mit nur einer Abflachung 8 gibt. Während der Umfangsrand des Hochdruckinjektionsspritzenkörpers 2 eine relativ geringe radiale Erstreckung hat, d.h. also ein verhältnismäßig schmaler Umfangsrand ist, besitzt, wie Figur 10 zeigt, der als Zylindergriffplatte vorgesehene Umfangsrand 6 eines Handinjektionsspritzenkörpers 4 diametral gegenüberliegende radiale Vorsprünge, die als Flügel 9 bezeichnet werden und eine relativ große axiale Erstreckung besitzen, welche im allgemeinen wesentlich größer als diejenige des Umfangsrands 3 des Hochdruckinjektionsspritzenkörpers 1 ist. Außerdem sind an dem Teil des Umfangsrandes 6, der sich zwischen den Flügeln 9 befindet, vorzugweise Abflachungen 6a, 6b vorgesehen.

Es sei nun eine bevorzugte Ausführungsform eines erfindungsgemäßen Umrüstsatzes zunächst anhand der Figur 1 beschrieben, die diesen Umrüstsatz eingebaut in einen Injektionsautomaten zeigt, von dem aus Gründen der vereinfachten Darstellung nur die kraftgetrieben ein- und ausfahrbare Spindelstange 10 dargestellt ist, die - im dargestellten Zustand des Injektionsautomaten - zum Verschieben des Kolbens 11 einer Handinjektionsspritze 12 dient, von der in Figur 1 nur der distale Endbereich gezeigt ist.

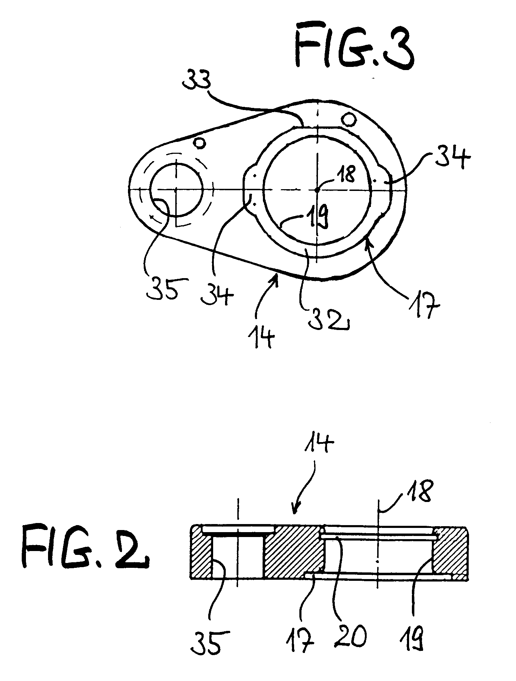

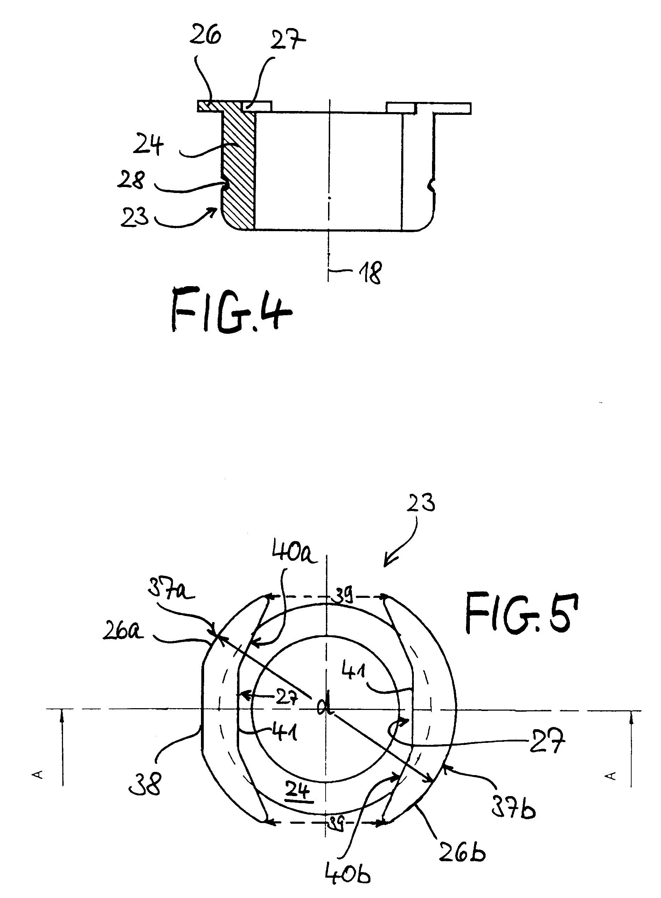

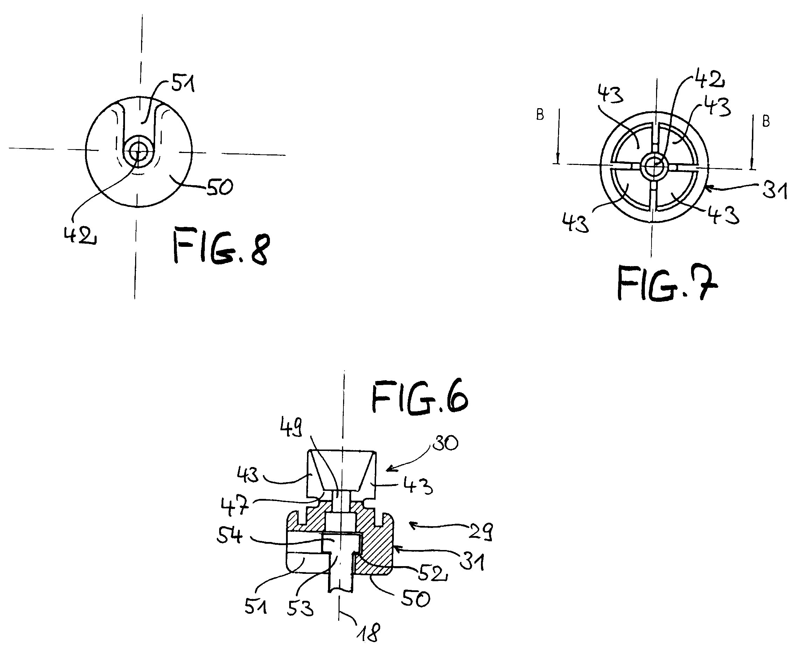

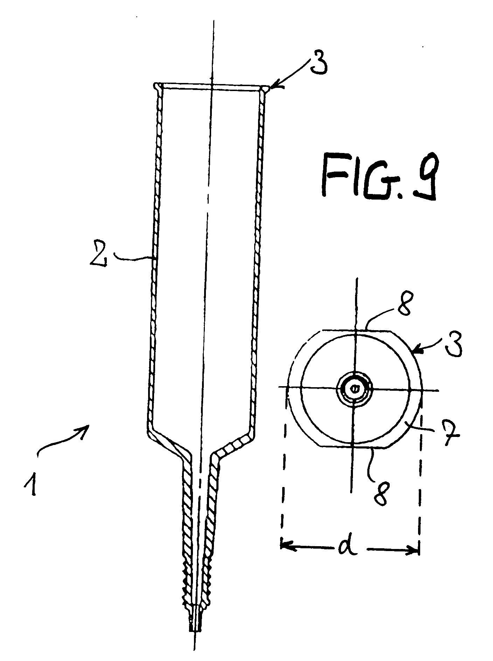

Der in Figur 1 gezeigte Umrüstsatz zum Umrüsten eines Injektionsautomaten für ein intravasales Injizieren aus Handinjektionsspritzen umfaßt einen Umrüstrezipienten 13, eine Reduzierhülse 23 und einen Umrüstkolbenaufnehmer 29, die wie folgt ausgebildet und in den Injektionsautomaten eingebaut sind:
(1) Der Umrüstrezipient 13 besitzt einen plattenförmigen Aufnahmekopf 14 und ein plattenförmiges Verschlußstück 15, die in der vorliegenden Ausführung durch eine in Bohrungen 35, 36 vorgesehene Schwenkachse 16 so verbunden sind, daß sie senkrecht zur Zeichnungsebene gegenseitig derart verschwenkbar sind, daß eine in der oberen Fläche des Aufnahmekopfs vorgesehene Ausnehmung 17 von dem Verschlußstück 15 völlig freigegeben wird. Anstelle dieser Art der bewegbaren Verbindung zwischen dem Aufnahmekopf 14 und dem Verschlußstück 15 kann auch jede Art einer anderen, an sich bekannten bewegbaren Verbindung vorgesehen sein, soweit es diese Verbindung gestattet, das Verschlußstück aus dem Bereich der Ausnehmung 17 herauszubewegen, z.B. eine Klappverbindung, eine Bajonettverbindung o.dgl. Der Aufnahmekopf 14 ist mit einer bezüglich der Achse 18 zylindrischen Aufnahmeführung 19 versehen, die beim Betrieb des Injektionsautomaten mit Hochdruckinjektionsspritzen den distalen Endbereich eines Hochdruckinjektionsspritzenkörpers 1 (siehe Figur 9) eng umschließt, also einen Durchmesser hat, der dem Außendurchmesser des Spritzenzylinders 2 der Hochdruckinjektionsspritze entspricht. In der Aufnahmeführung 19 ist weiterhin eine umlaufende Nut 20 ausgebildet, in welche ein O-Ring eingelegt ist, der einen in das Innere der Aufnahmeführung 19 hereinragenden elastischen Vorsprung 21 bildet. Schließlich weist das Verschlußstück 15 des Umrüstrezipienten 13 eine Bohrung 22 auf, deren Durchmesser gleich dem Durchmesser der Aufnahmeführung 19 ist. Dieser Umrüstrezipient 13 ist an dem Injektionsautomaten lösbar so anzubringen, daß eine in den Rezipienten eingespannte Hand- oder Hochdruckinjektionsspritze mittels der Spindelstange 10 betätigt werden kann.
(2) Die Reduzierhülse 23 ist zur Verkleinerung des effektiven Innendurchmessers der zylindrischen Aufnahmeführung 19 in letztere eingesetzt und umfaßt
   (i) einen im wesentlichen zylindrischen Hülsenkörper 24, der vorliegend, damit er möglichst leicht in die zylindrische Aufnahmeführung 19 einführbar ist, an seinem proximalen Ende 25 verjüngt ist, und zwar vorliegend durch Abrundung der äußeren Vorderkante; im übrigen entspricht der Außendurchmesser des Hülsenkörpers 24 im wesentlichen dem Innendurchmesser der Aufnahmeführung 19, während der Innendurchmesser des Hülsenkörpers 24 dem Außendurchmesser des Spritzenzylinders 5 (siehe auch Figur 10) einer Handinjektionsspritze vorbestimmten Nennvolumens entspricht; und
   (ii) einen Umfangsrand 26, der radial von dem distalen Ende der Reduzierhülse 23 vorsteht und in der Ausnehmung 17 des Aufnahmekopfes 14 aufgenommen ist; dieser radiale Umfangsrand, der in einer besonders bevorzugten Ausführungsform weiter unten in Verbindung mit den Figuren 4 und 5 näher erläutert ist, hat eine Ausnehmung 27, die so ausgebildet ist, daß sie den als Zylindergriffplatte vorgesehenen radialen Umfangsrand 6 einer Handinjektionsspritze aufnehmen kann, soweit sich dieser radial innerhalb des Bereichs des Umfangsrandes 26 befindet, d.h. bis auf die über den Umfangsrand 26 radial hinausragenden Endbereiche der Flügel 9 (siehe Figur 10). Die Flügel 9 der Handinjektionsspritze 12 verlaufen daher durch radiale Ausnehmungen im Umfangsrand 26 hindurch, so daß deren Endbereiche in entsprechenden Ausnehmungsbereichen 34 - in Figur 1 ebenfalls nicht sichtbar, aber in Verbindung mit Figur 3 weiter unten beschrieben - in der Ausnehmung 17 aufgenommen sind; demgemäß sind der Umfangsrand 26 der Reduzierhülse 23 und der als Zylindergriffplatte an der Handinjektionsspritze 12 vorgesehene Umfangsrand 6, soweit er über den Außendurchmesser der Reduzierhülse 23 vorsteht, beide vollständig in der Umfangsausnehmung 17 des Aufnahmekopfs 14 aufgenommen, wobei, wie bereits oben erwähnt, der übrige Teil des Umfangsrands 6 der Handinjektionsspritze 12, soweit er radial innerhalb des Durchmesserbereichs der Reduzierhülse 23 liegt, von der ihm entsprechenden Ausnehmung 27 am oberen Ende der Reduzierhülse 24 aufgenommen ist;
   (iii) eine um den Außenumfang des Hülsenkörpers 24 umlaufende Vertiefung 28, die vorliegend als im Querschnitt gerundete Rille ausgebildet ist und dazu dient, den in die Aufnahmeführung 19 vorstehenden Teil des elastischen Vorsprungs 20 aufzunehmen, so daß dadurch die Reduzierhülse 23 sicherer in der Aufnahmeführung 19 gehalten wird.
(3) Der Umrüstkolbenaufnehmer 29 ist mit einem Befestigungsmittel 30 zum lösbaren Befestigen desselben an dem freien Ende der Spindelstange 10 versehen und besitzt weiterhin einen mit diesem Befestigungsmittel 30 verbundenen Ankopplungskopf 31, der sowohl zum Ankoppeln des Kolbenaufnehmers 29 an den Kolben einer Hochdruckinjektionsspritze als auch zum Verschieben des Kolbens 11 der Handinjektionsspritze 12 durch Druckbeaufschlagung geeignet ist. Der - senkrecht zur Achse 18 definierte - Maximaldurchmesser D des Umrüstkolbenaufnehmers 29 ist gleich dem oder kleiner als der Außendurchmesser des Kolbens 11 der in die Reduzierhülse passenden Handinjektionsspritze 12; eine besonders bevorzugte Ausführungsform des Umrüstkolbenaufnehmers 29 ist weiter unten anhand der Figuren 6, 7 und 8 näher beschrieben.

Da die Ausnehmung 17 des Aufnahmekopfs 14 so ausgebildet ist, daß sie sowohl den radialen Umfangsrand 26 der Reduzierhülse 23 als auch die radialen Endbereiche der Flügel 9 einer Handinjektionsspritze 12 aufnehmen kann, und da die Ausnehmung 27 der Reduzierhülse 23 so ausgebildet ist, daß sie den radialen Umfangsrand 3 (siehe Figur 9) des Hochdruckinjektionsspritzenkörpers 1 aufnehmen kann, und da schließlich der Umrüstkolbenaufnehmer 29 so ausgebildet ist, daß sein Ankopplungskopf 31 an einem ein verdicktes Ende aufweisenden Zapfen 53, 54 (siehe Figur 6) des Kolbens einer Hochdruckinjektionsspritze ankoppelbar ist, so daß dieser Kolben durch die Spindelstange 10 sowohl ein- als auch ausgefahren werden kann, bedarf es nur der Herausnahme der Reduzierhülse 23 einschließlich des O-Rings 21 aus der in Figur 1 gezeigten Konfiguration, um den Injektionsautomaten für den Betrieb mit Hochdruckinjektionsspritzen verwendbar zu machen. Demgemäß braucht jeweils nur die Reduzierhülse 23 mit dem O-Ring eingesetzt oder entfernt zu werden, wenn der Injektionsautomat mit der jeweils anderen Injektionsspritzenart, nämlich Handinjektionsspritze oder Hochdruckinjektionsspritze, betrieben werden soll.

Es sei nun anhand der Figuren 2 und 3 eine Ausführungsform des Aufnahmekopfs 14 beschrieben, die in Figur 2 in einem durch die Achse 18 und die Mitte der Schwenkachse 16 hindurchgehenden Querschnitt und in Figur 3 in einer Aufsicht von - bezogen auf Figur 1 - oben gezeigt ist, soweit sich diese Ausbildung nicht schon aus der Beschreibung der Figur 1 ergibt, auf die ergänzend zu den nachstehenden Ausführungen verwiesen wird:

Wie aus Figur 3 ersichtlich ist, umfaßt die Ausnehmung 17 des Aufnahmekopfs 14 zwei Ausnehmungsbereiche, die zusammen eine durchgehende Gesamtausnehmung bilden und sich teilweise überschneiden, d.h. teilweise identisch sind:
(a) einen ersten Ausnehmungsbereich 32, der mit Ausnahme einer Abflachung 33 Kreisringform hat und zur drehfesten Aufnahme eines vom distalen Ende eines Hochdruckinjektionsspritzenkörpers 2 als Zylindergriffplatte vorstehenden Umfangsrands 3 (siehe Figur 9) dient, wobei die eine Abflachung 8 des Umfangsrands 3 formschlüssig mit der Abflachung 33 des ersten Ausnehmungsbereichs 32 in Eingriff tritt und so ein Verdrehen der Hochdruckinjektionsspritzen um deren Längsachse verhindert; und
(b) einen aus zwei Teilbereichen bestehenden zweiten Ausnehmungsbereich 34 für die radialen Endbereiche der Flügel 9 des als Zylindergriffplatte an einem Handinjektionsspritzenkörper 5 vorgesehenen Umfangsrands 6, wobei sich der zweite Ausnehmungsbereich 34 im Bereich der vorgenannten Flügel 9 mit dem ersten Ausnehmungsbereich 32 überschneidet.

Der Vollständigkeit halber ist zu erwähnen, daß der Aufnahmekopf 14 die obengenannte Bohrung 35 für die Aufnahme der Schwenkachse 16 besitzt, welche im zusammengebauten Zustand des Umrüstrezipienten 13 mit der entsprechenden Bohrung 36 in dem Verschlußstück 15 fluchtet.

Es sei nun anhand der Figuren 4 und 5 eine bevorzugte Ausführungsform einer Reduzierhülse beschrieben, die als die Reduzierhülse 23 in Figur 1 vorgesehen ist: Diese Reduzierhülse 23 wird hier nur noch hinsichtlich der Konfiguration ihres radialen Umfangsrands 26 erläutert, während im übrigen auf deren Beschreibung anhand der Figur 1 verwiesen wird. Der Umfangsrand 26 besteht vorliegend aus zwei Umfangsrandteilen 26a und 26b, die diametral entgegengesetzt von dem Umfang des Hülsenkörpers 24 vorstehen.

Die äußere Konfiguration 37a und 37b der Umfangsrandteile 26a und 26b entspricht der äußeren Konfiguration der diametral gegenüberliegenden Bereiche eines üblichen Umfangsrands 3 eines Hochdruckinjektionsspritzenkörpers 1 mit folgender Maßgabe:
(a) Die beiden diametral gegenüberliegenden Bereiche des zugrundegelegten Umfangsrands 3 des Hochdruckinjektionsspritzenkörpers 1 sind so gewählt, daß wenigstens einer eine Abflachung 8, vorzugsweise mittig, aufweist,
(b) es braucht nur einer der beiden Umfangsrandteile 26a, 26b eine der Abflachung 8 entsprechende Abflachung 38 aufzuweisen, wobei es aber auch möglich ist, daß beide Umfangsrandteile 26a und 26b je eine Abflachung 38 haben, und
(c) die Umfangsrandteile 26a und 26b erstrecken sich nur soweit um den Umfang der Reduzierhülse 23 herum, daß zwischen ihnen je eine Umfangsrandaussparung 39 bleibt, deren Weite der Breite der Flügel 9 einer zu der Reduzierhülse 23 passenden Handinjektionsspritze 12 an dieser Stelle entspricht.

Die innere Konfiguration 40a und 40b der Umfangsrandteile 26a und 26b ist so gestaltet, daß sie der äußeren Konfiguration des als Zylindergriffplatte vorgesehenen Umfangsrands 6 der Handinjektionsspritze 12 entspricht, soweit dieser Umfangsrand nicht über den äußeren Durchmesser d des Umfangsrands 3 einer üblichen Hochdruckinjektionsspritze vorsteht, sich also zwischen den beiden Umfangsrandteilen 26a und 26b befindet. Wie Figur 5 zeigt, umfaßt die innere Konfiguration 40a und 40b jeweils eine Abflachung 41, die je einer der Abflachungen 6a und 6b des Umfangsrands 6 (siehe Figur 10) einer Handinjektionsspritze 12 entspricht.

Auf diese Weise wird erreicht, daß in der bevorzugten Ausführungsform der Reduzierhülse 23 einerseits deren Umfangsrand 26 außen formschlüssig von der Ausnehmung 17 des Aufnahmekopfs 14 aufgenommen wird, und daß andererseits der Umfangsrand 26 zusammen mit der Ausnehmung 17 des Aufnahmekopfs 14 den Umfangsrand 6 einer Handinjektionsspritze 12 formschlüssig aufnimmt.

Es sei nun anhand der Figuren 6, 7 und 8 schließlich noch eine besonders bevorzugte Ausführungsform eines Umrüstkolbenaufnehmers beschrieben, der als der Umrüstkolbenaufnehmer 29 in Figur 1 vorgesehen ist, wobei ergänzend zu der nachfolgenden Erläuterung auf die Beschreibung anhand der Figur 1 verwiesen wird:
(a) Das Befestigungsmittel 30 (vollständig in Figur 1 gezeigt) ist als Einspannvorrichtung zum radialen Einspannen desselben in eine in dem freien Ende der Spindelstange vorgesehene zylindrische Bohrung 42 ausgebildet, indem um die Achse 18 herum vier Segmente 43 eines Konuskörpers angeordnet sind, durch deren Basis 47 eine Bohrung 49 hindurchgeht. Innerhalb dieser Segmente 43 ist ein entsprechender Konus 46 vorgesehen, durch den eine Gewindebohrung 44 hindurchgeht, die der Bohrung 49 entspricht und durch die von unten her eine Schraube 48 in die Gewindebohrung 44 eingeschraubt wird, während von oben her in letztere ein Gewindezapfen 45 der Spindelstange 10 eingeschraubt wird, so daß die Segmente 43 durch den Konus 46 nach auswärts gedrückt und gegen den Innenumfang der Bohrung 49 verspannt werden.
   Die Segmente 43 sind einstückig mit der Basis 47 und damit mit dem Ankopplungskopf 31, oder die Basis 47 ist fest mit dem Ankopplungskopf 31 verbunden.
(b) Der Ankopplungskopf 31, dessen Durchmesser D gleich dem oder kleiner als der Durchmesser des Kolbens 11 der Handinjektionsspritze 12 ist, hat an seinem dem Befestigungsmittel 30 abgewandten Ende eine flache Druckfläche 50 zum Eingriff mit der flachen Rückseite des Kolbens 11, um diesen zum Zwecke der Injektion zu verschieben. Außerdem ist in dem Ankopplungskopf 31 eine schlitzförmige, sich radial erstreckende Vertiefung 51 vorgesehen, die eine Hinterschneidung 52 hat, so daß darin ein Zapfen 53 mit einem verdickten Ende 54 lose aufgenommen werden kann, wie er an der rückwärtigen Kolbenfläche des Kolbens einer Hochdruckinjektionsspritze vorgesehen ist. Auf diese Weise kann der Kolben einer solchen Hochdruckinjektionsspritze mittels der kraftgetriebenen Spindelstange 10 sowohl in einem Saughub zum Füllen der Hochdruckinjektionsspritze als auch in einem Druckhub zum Injizieren aus der Hochdruckinjektionsspritze betätigt werden.

## Patentansprüche

1. Umrüstsatz für einen Injektionsautomaten zum intravasalen Injizieren von parenteralen Lösungen aus Hochdruckinjektionsspritzen (1), wobei der umzurüstende Injektionsautomat folgendes aufweist:
(a) einen Rezipienten (13) zum lösbaren Festhalten einer Hochdruckinjektionsspritze (1), der einen Aufnahmekopf (14) besitzt, welcher mit einer zylindrischen Aufnahmeführung zum engen Umschließen des distalen Endbereichs des Hochdruckinjektionsspritzenkörpers (2) und mit einer radialen Ausnehmung für die im wesentlichen drehfeste Aufnahme eines von dem distalen Ende des Hochdruckinjektionsspritzenkörpers (2) vorstehenden Umfangsrandes (3) versehen ist; und
(b) eine kraftgetrieben ein- und ausfahrbare Spindelstange (10), die zum Verschieben des Kolbens der Hochdruckinjektionsspritze (1) an ihrem freien Ende einen lösbar daran befestigten Kolbenaufnehmer aufweist, welcher einen Ankopplungskopf zum Ankoppeln desselben an den Kolben der Hochdruckinjektionsspritze (1) hat,
dadurch **gekennzeichnet,** daß der Umrüstsatz zum Umrüsten des Injektionsautomaten für den Gebrauch mit sowohl Hochdruckinjektionsspritzen als auch Handinjektionsspritzen (12), bei denen der maximale Durchmesser des als Zylindergriffplatte vorgesehenen distalen Umfangsrandes (6) gleich dem oder kleiner als der Durchmesser der Aufnahmeführung (19) ist, folgendes umfaßt:
(1) wenigstens eine Reduzierhülse (23) zur Verkleinerung des effektiven Innendurchmessers der zylindrischen Aufnahmeführung (19), umfassend
(i) einen im wesentlichen zylindrischen Hülsenkörper (24), dessen Außendurchmesser dem Innendurchmesser der Aufnahmeführung (19) entspricht und dessen Innendurchmesser dem Außendurchmesser des Handinjektionsspritzenkörpers (5) entspricht, und
(ii) einen Umfangsrand (26) am distalen Ende der Reduhülse, welcher, soweit er von dem Hülsenkörper (24) radial vorsteht, in der Ausnehmung (17) des Aufnahmekopfes (19) aufnehmbar ist und der eine Ausnehmung (27) hat, in welcher der mit dem Umfangsrand (26) der Reduzierhülse (23) koextensive Bereich des distalen Umfangsrands (6) der Handinjektionsspritze aufnehmbar ist; und
(2) wenigstens einen Umrüstkolbenaufnehmer (29), dessen radialer Maximaldurchmesser (D) gleich dem oder kleiner als der Durchmesser des Kolbens (11) der Handinjektionsspritze (12) ist, und der mit einem Befestigungsmittel (30) zum lösbaren Befestigen desselben an dem freien Ende der Spindelstange (10) sowie mit einem Ankopplungskopf (31) zum Ankoppeln des Kolbenaufnehmers (29) an den Kolben einer Hochdruckinjektionsspritze und zum Drücken auf den Kolben (11) der Handinjektionsspritze (12) versehen ist.

2. Umrüstsatz nach Anspruch 1 oder nach dem Oberbegriff des Anspruchs 1, dadurch **gekennzeichnet,** daß der Umrüstsatz zum Umrüsten des Injektionsautomaten für den Gebrauch mit sowohl Hochdruckinjektionsspritzen als auch Handinjektionsspritzen (12), bei denen der maximale Durchmesser des als Zylindergriffplatte vorgesehenen Umfangsrandes (6) größer als der Durchmesser der Aufnahmeführung (19) ist, wenigstens einen Umrüstaufnahmekopf (17) oder wenigstens einen Umrüstrezipienten (13) umfaßt, wobei die radiale Ausnehmung (17) des Umrüstaufnahmekopfes (14) außer einem ersten Ausnehmungsbereich (32) für die Aufnahme des von dem distalen Ende eines Hochdruckinjektionsspritzenkörpers (2) vorstehenden Umfangsrandes (3), einen zweiten Ausnehmungsbereich (34) für die radialen Endbereiche der Flügel (9) der Handinjektionsspritze (12) aufweist.

3. Umrüstsatz nach Anspruch 1, **gekennzeichnet** durch wenigstens einen Umrüstaufnahmekopf (14) oder wenigstens einen Umrüstrezipienten (13) mit einem Umrüstaufnahmekopf (14), für das distale Ende einer Hochdruckinjektionsspritze, wobei die Aufnahmeführung (19) des Umrüstaufnahmekopfs mit einem vorzugsweise um den inneren Umfang derselben umlaufenden, elastischen Vorsprung (21) versehen ist und die Reduzierhülse (23) auf deren Außenumfang eine dem Vorsprung (21) entsprechende Vertiefung aufweist.

4. Umrüstsatz nach Anspruch 2, dadurch **gekennzeichnet,** daß die Aufnahmeführung (19) mit einem vorzugsweise um den inneren Umfang derselben umlaufenden, elastischen Vorsprung (21) versehen ist und die Reduzierhülse (23) auf deren Außenumfang eine dem Vorsprung (21) entsprechende Vertiefung (28) aufweist.

5. Umrüstsatz nach Anspruch 3 oder 4, dadurch **gekennzeichnet,** daß der elastische Vorsprung (21) von einem O-Ring gebildet ist, der in eine auf den Innenumfang der Aufnahmeführung (19) ausgebildete Nut (20) eingelegt ist.

6. Umrüstsatz nach Anspruch 2 oder 3, dadurch **gekennzeichnet,** daß die Konfiguration der Ausnehmung (17) des Umrüstaufnahmekopfs (14) bzw. des ersten Ausnehmungsbereichs (32) so ausgebildet ist, daß er den Umfangsrand (3) des Hochdruckinjektionsspritzenkörpers (2) drehfest oder im wesentlichen drehfest, vorzugsweise formschlüssig, aufnimmt.

7. Umrüstsatz nach einem der Ansprüche 1 bis 6, dadurch **gekennzeichnet,** daß die Konfiguration des Umfangsrands (26) der Reduzierhülse (23) so ausgebildet ist, daß die Reduzierhülse (23) drehfest oder im wesentlichen drehfest in der Ausnehmung (17) des Aufnahmekopfes oder des Umrüstaufnahmekopfes (14) aufgenommen wird.

8. Umrüstsatz nach Anspruch 7, dadurch **gekennzeichnet,** daß die Konfiguration des Umfangsrandes (26) der Reduzierhülse (23) mit Ausnahme von gegebenenfalls vorgesehenen Umfangsaussparungen (39) für die Flügel (9) der Handinjektionsspritze (12) der Konfiguration des Umfangsrandes (3) einer Hochdruckinjektionsspritze entspricht.

9. Umrüstsatz nach einem der Ansprüche 1 bis 8, dadurch **gekennzeichnet,** daß die Ausnehmung (27) des Umfangsrandes der Reduzierhülse (23) den damit koextensiven Bereich des Umfangsrands (6) der Handinjektionsspritze (12) drehfest oder im wesentlichen drehfest, vorzugsweise formschlüssig, aufnimmt.

10. Umrüstsatz nach einem der Ansprüche 2 bis 9, dadurch **gekennzeichnet,** daß er für HandinjektionsSpritzen (12) von 30 ml und 50 ml und HochdruckinjektionsSpritzen von 50 bis 200 ml ausgebildet ist.

11. Umrüstsatz nach einem der Ansprüche 1 bis 9, dadurch **gekennzeichnet,** daß er für alle oder einem Teil der Handinjektionsspritzen (12) von 1 bis 50 ml und Hochdruckinjektionsspritzen von 50 bis 200 ml ausgebildet ist.

12. Umrüstsatz nach einem der Ansprüche 1 bis 11, dadurch **gekennzeichnet,** daß er mehrere Umrüstrezipienten (13) oder Umrüstaufnahmeköpfe (14) und/oder mehrere Reduzierhülsen (23) und/oder mehrere Umrüstkolbenaufnehmer (29) umfaßt.

## Claims

1. Conversion kit for an injector for intravascular injection of parenteral solutions from high-pressure syringes, the injector to be converted comprising the following:
(a) a receiver (13) for releasably retaining a high-pressure syringe (1), said receiver having a receiving head (14) provided with a cylindrical receiving guide for tightly surrounding the distal end region of the high-pressure syringe body (2) and with a radial recess for substantially non-rotary reception of a peripheral edge (3) projecting from the distal end of the high-pressure syringe body (2), and
(b) a power-driven retractable and extensible spindle rod (10) having a plunger receptacle releasably secured to its free end for displacement of the plunger of the high-pressure syringe (1), the plunger receptacle having a coupling head for coupling it to the plunger of the high-pressure syringe (1),
characterised in that for the purpose of converting the injector for use with both high-pressure syringes and manual syringes (12) in which the maximum diameter of the distal peripheral edge (6), provided in the form of a cylindrical gripping plate, is equal to or less than the diameter of the receiving guide (19) the conversion kit comprises the following:
(1) at least one reducing sleeve (23) for reducing the effective internal diameter of the cylindrical receiving guide (19), comprising
(i) a substantially cylindrical sleeve body (24), the external diameter of which corresponds to the internal diameter of the receiving guide (19) and the internal diameter of which corresponds to the external diameter of the manual-syringe body (5), and
(ii) a peripheral edge (26) at the distal end of the reducing sleeve, said peripheral edge being capable, to the extent that it projects radially from the sleeve body (24), of being received in the recess (17) in the receiving head (19) and having a recess (27) in which that region of the distal peripheral edge (6) of the manual syringe which is co-extensive with the peripheral edge (26) of the reducing sleeve (23) is capable of being received; and
(2) at least one convertible plunger receptacle (29), the radial maximum diameter (D) of which is equal to or less than the diameter of the plunger (11) of the manual syringe (12), said convertible plunger receptacle being provided with a securing means (30) for releasably securing it to the free end of the spindle rod (10) and also with a coupling head (31) for coupling the plunger receptacle (29) to the plunger of a high-pressure syringe and for exerting pressure on the plunger (11) of the manual syringe (12).

2. Conversion kit according to Claim 1 or according to the preamble to Claim 1, characterised in that the conversion kit for converting the injector for use with both high-pressure syringes and manual syringes (12) in which the maximum diameter of the peripheral edge (6), provided in the form of a cylindrical gripping plate, is greater than the diameter of the receiving guide (19) comprises at least one convertible receiving head (17) or at least one convertible receiver (13), the radial recess (17) in the convertible receiving head (14) comprising, besides a first recessed region (32) for receiving the peripheral edge (3) projecting from the distal end of a high-pressure syringe body (2), a second recessed region (34) for the radial end regions of the wings (9) of the manual syringe (12).

3. Conversion kit according to Claim 1, characterised by at least one convertible receiving head (14) or at least one convertible receiver (13) with a convertible receiving head (14) for the distal end of a high-pressure syringe, the receiving guide (19) of the convertible receiving head (14) being provided with a resilient projection (21) preferably extending around its internal periphery, and the reducing sleeve (23) comprising on its external periphery a depression corresponding to the projection (21).

4. Conversion kit according to Claim 2, characterised in that the receiving guide (19) is provided with a resilient projection (21) preferably extending around its internal periphery, and the reducing sleeve (23) comprises on its external periphery a depression (28) corresponding to the projection (21).

5. Conversion kit according to Claim 3 or 4, characterised in that the resilient projection (21) is constituted by an O-ring inserted into a groove (20) which is formed on the internal periphery of the receiving guide (19).

6. Conversion kit according to Claim 2 or 3, characterised in that the configuration of the recess (17) in the convertible receiving head (14) or in the first recessed region (32) is such that it non-rotatably or substantially non-rotatably, preferably positively, receives the peripheral edge (3) of the high-pressure syringe body (2).

7. Conversion kit according to any of Claims 1 to 6, characterised in that the configuration of the peripheral edge (26) of the reducing sleeve (23) is such that the reducing sleeve (23) is non-rotatably or substantially non-rotatably received in the recess (17) in the receiving head or of the convertible receiving head (14).

8. Conversion kit according to Claim 7, characterised in that the configuration of the peripheral edge (26) of the reducing sleeve (23) corresponds to the configuration of the peripheral edge (3) of a high-pressure syringe, with the exception of peripheral recesses (39) which are provided as appropriate for the wings (9) of the manual syringe (12).

9. Conversion kit according to any of Claims 1 to 8, characterised in that the recess (27) in the peripheral edge of the reducing sleeve (23) non-rotatably or substantially non-rotatably, preferably positively, receives the co-extensive region of the peripheral edge (6) of the manual syringe (12).

10. Conversion kit according to any of Claims 2 or 9, characterised in that it is designed for 30-ml and 50-ml manual syringes (12) and for 50-ml to 200-ml high-pressure syringes.

11. Conversion kit according to any of Claims 1 to 9, characterised in that it is designed for all or some 1-ml to 50-ml manual syringes (12) and for 50-ml to 200-ml high-pressure syringes.

12. Conversion kit according to any of Claims 1 to 11, characterised in that it comprises several convertible receivers (13) or convertible receiving heads (14) and/or several reducing sleeves (23) and/or several convertible plunger receptacles (29).

## Revendications

1. Ensemble de conversion pour un système automatique d'injection permettant l'injection intravasculaire de solutions parentérales avec des seringues d'injection à haute pression (1) dans lequel le système d'injection devant être converti possède ce qui suit :
a) un récipient (13) servant à fixer de façon amovible une seringue d'injection à haute pression (1), et qui possède une tête de réception (14), qui comporte un guide cylindrique de réception servant à entourer étroitement la partie d'extrémité distale du corps (2) de la seringue d'injection à haute pression, et un évidement radial servant à loger, d'une manière essentiellement bloquée contre toute rotation, un bord circonférentiel (3) gui fait saillie à partir de l'extrémité distale du corps (2) de la seringue d'injection à haute pression ; et
b) une tige de broche (10), qui peut être rétractée et ressortie sous l'action d'une force et qui possède, au niveau de son extrémité libre, pour le déplacement du piston de la seringue d' injection à haute pression (1), un logement de piston fixé de façon amovible sur cette extrémité et gui possède une tête d'accouplement permettant son accouplement au piston de la seringue d'injection à haute pression (1),
caractérisé en ce que l'ensemble de conversion servant à convertir le système automatique d'injection pour son utilisation aussi bien avec des seringues d'injection à haute pression qu'avec des seringues d'injection manuelle (12), dans lesquelles le diamètre maximum du bord circonférentiel distal (6), qui est prévu sur la plaque d'appui du cylindre, est égal ou inférieur au diamètre du guide de réception (19), comprend ce qui suit :
(1) au moins une douille de réduction (23) servant à réduire le diamètre intérieur effectif du guide de réception cylindrique (19), comprenant :
(i) un corps de douille essentiellement cylindrique (24), dont le diamètre extérieur correspond au diamètre intérieur du guide de réception (19), et dont le diamètre intérieur correspond au diamètre extérieur du corps (5) de la seringue d'injection manuelle,
(ii) un bord circonférentiel (26) situé sur l'extrémité distale de la douille de réduction et qui, dans la mesure où il fait saillie radialement à partir du corps (24) de la douille, peut être logé dans l'évidement (17) de la tête de réception (19) et possède un évidement (24) dans lequel peut être logée la partie du bord circonférentiel distal (6) de la seringue d'injection manuelle, qui s'étend sur une même distance que le bord circonférentiel (26) de la douille de réduction (23) ; et
(2) au moins un logement (29) pour le piston de conversion, dont le diamètre radial maximum (B) est égal ou inférieur au diamètre du piston (11) de la seringue d'injection manuelle (12), et qui comporte un moyen de fixation (30) utilisé pour sa fixation amovible sur l'extrémité libre de la tige de broche (10), ainsi qu'une tête d'accouplement (31) pour l'accouplement du logement de piston (29) au piston d'une seringue d'injection à haute pression et pour l'application d'une pression au piston (11) de la seringue d'injection manuelle (12).

2. Ensemble de conversion selon la revendication 1 ou selon le préambule de la revendication 1, caractérisé en ce que pour la conversion du système automatique d'injection pour l'utilisation aussi bien avec des seringues d'injection à haute pression qu'avec des seringues d'injection manuelle (12), dans lesquelles le diamètre maximum du bord périphérique (6) prévu en tant que plaque d'appui du cylindre est supérieur au diamètre du guide de logement (19), l'ensemble de conversion comprend au moins une tête de réception de conversion (17) ou au moins un récipient de conversion (13), l'évidement radial (17) de la tête de réception de conversion (14) possédant, pour la réception du bord périphérique (3) qui fait saillie à partir de l'extrémité distale d'un corps (2) de la seringue d'injection à haute pression, une seconde partie d'évidement (34) pour les parties d'extrémité radiales des ailes (9) de la seringue d'injection manuelle (12).

3. Ensemble de conversion selon la revendication 1, caractérisé par au moins une tête de réception de conversion (14) ou par au moins un récipient de conversion (13) comprenant une tête de réception de conversion (14), pour l'extrémité distale d'une seringue d'injection à haute pression, et dans lequel le guide de réception (19) de la tête de réception de conversion comporte une partie saillante élastique (21) qui s'étend circonférentiellement de préférence autour de la circonférence intérieure de cette tête, et la douille de réduction (23) possède, sur sa périphérie extérieure, un renfoncement correspondant à la partie saillante (21).

4. Ensemble de conversion selon la revendication 2, caractérisé en ce que le guide de réception (19) est équipé d'une partie saillante élastique (21), qui entoure de préférence la périphérie intérieure de ce guide, et la douille de réception (23) comporte, sur sa périphérie extérieure, un renfoncement (28) qui correspond à la partie saillante (21).

5. Ensemble de conversion selon la revendication 3 ou 4, caractérisé en ce que la partie saillante élastique (21) est formée par un joint torique, qui est inséré dans une gorge (20) aménagée dans la périphérie intérieure du guide de réception (19).

6. Ensemble de conversion selon la revendication 2 ou 3, caractérisé en ce que la configuration de l'évidement (17) de la tête de réception de conversion (14) ou de la première partie d'évidement (32) est agencée de telle sorte que cet élément loge le bord circonférentiel (3) du corps (2) de la seringue d'injection à haute pression d'une manière bloquée en rotation ou sensiblement bloquée en rotation, de préférence selon une liaison par formes complémentaires.

7. Ensemble de conversion selon l'une des revendications 1 à 6, caractérisé en ce que la configuration du bord périphérique (26) de la douille de réduction (23) est agencée de telle sorte que la douille de réduction (23) est logée en étant bloquée en rotation ou sensiblement bloquée en rotation dans l'évidement (17) de la tête de réception ou de la tête de réception de conversion (14).

8. Ensemble de conversion selon la revendication 7, caractérisé en ce que la configuration du bord circonférentiel (26) de la douille de réduction (23) correspond, à l'exception d'évidements circonférentiels éventuellement prévus (39) pour les ailes (9) de la seringue d'injection manuelle (12), à la configuration du bord périphérique (3) d'une seringue d'injection à haute pression.

9. Ensemble de conversion selon l'une des revendications 1 à 8, caractérisé en ce que l'évidement (27) du bord périphérique de la douille de réduction (23) loge la partie, qui s'étend sur la même distance que cette douille, du bord périphérique (6) de la seringue d'injection manuelle (12) de manière bloquée en rotation ou sensiblement bloquée en rotation, de préférence selon une liaison par formes complémentaires.

10. Ensemble de conversion selon l'une des revendications 2 à 9, caractérisé en ce qu'il est agencé pour des seringues d'injection manuelle (12) de 30 ml et de 50 ml et pour des seringues d'injection à haute pression de 50 à 200 ml.

11. Ensemble de conversion selon l'une des revendications 1 à 9, caractérisé en ce qu'il est agencé pour la totalité ou une partie des seringues d'injection manuelle (12) de 1 à 50 ml et des seringues d'injection à haute pression de 50 à 200 ml.

12. Ensemble de conversion selon l'une des revendications 1 à 11, caractérisé en ce qu'il comporte plusieurs récipients de conversion (13) ou têtes de réception de conversion (14) et/ou plusieurs douilles de réduction (23) et/ou plusieurs logements (22) pour pistons de conversion.
